(19) Europäisches Patentamt
European Patent Office
Office européen des brevets

(11) EP 3 154 985 B1

(12) **EUROPEAN PATENT SPECIFICATION**

(45) Date of publication and mention of the grant of the patent:
**06.06.2018 Bulletin 2018/23**

(21) Application number: **15745599.9**

(22) Date of filing: **09.06.2015**

(51) Int Cl.:
***C07D 493/06*** *(2006.01)* ***A61K 31/4178*** *(2006.01)*
***A61P 31/14*** *(2006.01)*

(86) International application number:
**PCT/US2015/034823**

(87) International publication number:
**WO 2015/191526 (17.12.2015 Gazette 2015/50)**

(54) **ANTIVIRAL COMPOUNDS**

ANTIVIRALE VERBINDUNGEN

COMPOSÉS ANTIVIRAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2014 US 201462011155 P**

(43) Date of publication of application:
**19.04.2017 Bulletin 2017/16**

(73) Proprietor: **Gilead Sciences, Inc.**
**Foster City, CA 94404 (US)**

(72) Inventors:
- **BACON, Elizabeth M.**
  **Foster City, CA 94404 (US)**
- **COTTELL, Jeromy J.**
  **Foster City, CA 94404 (US)**
- **LINK, John O.**
  **Foster City, CA 94404 (US)**
- **TREJO MARTIN, Teresa Alejandra**
  **Foster City, CA 94404 (US)**
- **ZIPFEL, Sheila**
  **Foster City, CA 94404 (US)**

(74) Representative: **Fairbairn, Angus Chisholm**
**Marks & Clerk LLP**
**90 Long Acre**
**London WC2E 9RA (GB)**

(56) References cited:
**WO-A1-2012/021591** **WO-A1-2012/109080**
**WO-A2-2012/068234**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## BACKGROUND

Hepatitis C is recognized as a chronic viral disease of the liver which is characterized by liver disease. Although drugs targeting the liver are in wide use and have shown effectiveness, toxicity and other side effects have limited their usefulness. Inhibitors of hepatitis C virus (HCV) are useful to limit the establishment and progression of infection by HCV as well as in diagnostic assays for HCV.

There is a need for new HCV therapeutic agents. In particular, there is a need for HCV therapeutic agents that have broad activity against HCV genotypes (e.g. genotypes 1, 2. 3, 4, 5, 6, etc.). There is also a particular need for agents that are less susceptible to viral resistance. Resistance mutations to inhibitors have been described for HCV NS5A for genotypes 1a and 1b in Antimicrobial Agents and Chemotherapy, September 2010, Volume 54, p. 3641-3650.

## SUMMARY

The present disclosure provides compounds for use in pharmaceutical compositions and methods for treating hepatitis C (HCV).

In one embodiment the disclosure provides the three compounds as depicted in claim 1, in the following also called compounds of formula (I), or pharmaceutically acceptable salts thereof.

The disclosure also provides isotopically enriched compounds that are compounds as described herein that comprise an enriched isotope at one or more positions in the compound.

The present disclosure also provides a pharmaceutical composition comprising a compound as described herein or a pharmaceutically acceptable salt thereof and at least one pharmaceutically acceptable carrier.

The present disclosure also provides a pharmaceutical composition for use in treating hepatitis C (HCV) or a hepatitis C associated disorder. In one embodiment the composition comprises at least one additional therapeutic agent for treating HCV. In one embodiment, the therapeutic agent is selected from ribavirin, an NS3 protease inhibitor, an inhibitor of HCV NS5B polymerase (e.g. nucleoside or nucleotide inhibitor of HCV NS5B polymerase), an alpha-glucosidase 1 inhibitor, a hepatoprotectant, a non-nucleoside inhibitor of HCV polymerase, or combinations thereof. In one embodiment, the composition further comprises an inhibitor of HCV NS5B polymerase. In one embodiment, the inhibitor of HCV NS5B polymerase is selected from ribavirin, viramidine, levovirin, a L-nucleoside, or isatoribine. In one embodiment, the inhibitor of HCV NS5B polymerase is sofosbuvir.

In one embodiment, provided is a pharmaceutical composition comprising a compound as described herein and at least one inhibitor of HCV NS5B polymerase, and at least one pharmaceutically acceptable carrier. In one embodiment, the composition further comprises an interferon, a pegylated interferon, ribavirin or combinations thereof. In one embodiment, the inhibitor of HCV NS5B polymerase is sofosbuvir.

In one embodiment, provided is a pharmaceutical composition comprising a compound as described herein and at least one NS3 protease inhibitor, and at least one pharmaceutically acceptable carrier. In one embodiment, the composition further comprises an inhibitor of HCV NS5B polymerase.

The present disclosure also provides a pharmaceutical composition further comprising an interferon or pegylated interferon.

The present disclosure also provides a pharmaceutical composition further comprising a nucleoside analog.

The present disclosure also provides for a pharmaceutical composition wherein said nucleoside analogue is selected from ribavirin, viramidine, levovirin, an L-nucleoside, and isatoribine and said interferon is $\alpha$-interferon or pegylated $\alpha$-interferon.

The present disclosure also provides for a method of treating a patient infected with hepatitis C virus, said method comprising administering to a human patient a compound described herein or a pharmaceutical composition as described herein.

The present disclosure also provides a method of inhibiting HCV, comprising administering to a mammal afflicted with a condition associated with HCV activity, an amount of a compound as described herein, effective to inhibit HCV.

The present disclosure also provides a compound as described herein for use in medical therapy (e.g. for use in inhibiting HCV activity or treating a condition associated with HCV activity), as well as the use of a compound as described herein for the manufacture of a medicament useful for inhibiting HCV or the treatment of a condition associated with HCV activity in a mammal.

The present disclosure also provides synthetic processes and novel intermediates disclosed herein which are useful for preparing compounds as described herein. Some of the compounds are useful to prepare other compounds as described herein.

In another aspect the disclosure provides a compound as described herein, or a pharmaceutically acceptable salt thereof, for use in the prophylactic or therapeutic treatment of hepatitis C or a hepatitis C associated disorder.

In another aspect the disclosure provides a method of inhibiting HCV activity in a sample comprising treating the sample with a compound as described herein.

Compounds of formula (I) have been found to possess useful activity against several HCV genotypes. Additionally certain compounds of formula (I) exhibit significant potency against resistant variants in, e.g., GT1.

## DETAILED DESCRIPTION

The term "treatment" or "treating," to the extent it relates to a disease or condition includes preventing the disease or condition from occurring, inhibiting the disease or condition, eliminating the disease or condition, and/or relieving one or more symptoms of the disease or condition.

The compounds described herein can also exist as tautomeric isomers in certain cases. Although only one tautomer may be depicted, all such forms are contemplated within the scope of the disclosure. For example, ene-amine tautomers can exist for purine, pyrimidine, imidazole, guanidine, amidine, and tetrazole systems and all their possible tautomeric forms are within the scope of the disclosure.

### *Salts and Hydrates*

Examples of physiologically or pharmaceutically acceptable salts of the compounds described herein include salts derived from an appropriate base, such as, for example, an alkali metal (for example, sodium), an alkaline earth metal (for example, magnesium), ammonium and $NX_4^+$ (wherein X is $C_1$-$C_4$ alkyl). Physiologically acceptable salts of a hydrogen atom or an amino group include salts of organic carboxylic acids such as, for example, acetic, benzoic, lactic, fumaric, tartaric, maleic, malonic, malic, isethionic, lactobionic and succinic acids; organic sulfonic acids, such as, for example, methanesulfonic, ethanesulfonic, benzenesulfonic and p-toluenesulfonic acids; and inorganic acids, such as, for example, hydrochloric, sulfuric, phosphoric and sulfamic acids. Physiologically acceptable salts of a compound of a hydroxy group include the anion of said compound in combination with a suitable cation such as, for example, $Na^+$ and $NX_4^+$ (wherein X is independently selected from H or a $C_1$-$C_4$ alkyl group).

For therapeutic use, salts of active ingredients of the compounds described herein will typically be physiologically acceptable, *i.e.* they will be salts derived from a physiologically acceptable acid or base. However, salts of acids or bases which are not physiologically acceptable may also find use, for example, in the preparation or purification of a physiologically acceptable compound. All salts, whether or not derived form a physiologically acceptable acid or base, are within the scope of the present disclosure.

Metal salts typically are prepared by reacting the metal hydroxide with a compound of this disclosure. Examples of metal salts which are prepared in this way are salts containing $Li^+$, $Na^+$, and $K^+$. A less soluble metal salt can be precipitated from the solution of a more soluble salt by addition of the suitable metal compound.

In addition, salts may be formed from acid addition of certain organic and inorganic acids, *e.g.,* HCl, HBr, $H_2SO_4$, $H_3PO_4$ or organic sulfonic acids, to basic centers, typically amines, or to acidic groups. Finally, it is to be understood that the compositions herein comprise compounds described herein in their un-ionized, as well as zwitterionic form, and combinations with stoichiometric amounts of water as in hydrates.

Also included within the scope of this disclosure are the salts of the parental compounds with one or more amino acids. Any of the natural or unnatural amino acids are suitable, especially the naturally-occurring amino acids found as protein components, although the amino acid typically is one bearing a side chain with a basic or acidic group, e.g., lysine, arginine or glutamic acid, or a neutral group such as, for example, glycine, serine, threonine, alanine, isoleucine, or leucine.

## Methods of Inhibition of HCV

The application describes methods of inhibiting the activity of HCV comprising the step of treating a sample suspected of containing HCV with a compound or composition as described herein.

The treating step comprises adding the compound described herein to the sample or it comprises adding a precursor of the composition to the sample. The addition step comprises any method of administration as described above.

If desired, the activity of HCV after application of the compound can be observed by any method including direct and indirect methods of detecting HCV activity. Quantitative, qualitative, and semiquantitative methods of determining HCV activity are all contemplated. Typically one of the screening methods described above are applied, however, any other method such as, for example, observation of the physiological properties of a living organism are also applicable.

Many organisms contain HCV. The compounds of this disclosure are useful in the treatment or prophylaxis of conditions associated with HCV activation in animals or in man.

However, in screening compounds capable of inhibiting HCV activity it should be kept in mind that the results of enzyme assays may not always correlate with cell culture assays. Thus, a cell based assay should typically be the

primary screening tool.

**Pharmaceutical Formulations**

The compounds of this disclosure are formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. All formulations will optionally contain excipients such as, for example, those set forth in the Handbook of Pharmaceutical Excipients (1986). Excipients include ascorbic acid and other antioxidants, chelating agents such as, for example, EDTA, carbohydrates such as, for example, dextrin, hydroxyalkylcellulose, hydroxyalkylmethylcellulose, stearic acid and the like. The pH of the formulations ranges from about 3 to about 11, but is ordinarily about 7 to 10. Typically, the compound will be administered in a dose from 0.01 milligrams to 2 grams. In one embodiment, the dose will be from about 10 milligrams to 450 milligrams. It is contemplated that the compound may be administered once, twice or three times a day.

While it is possible for the active ingredients to be administered alone it may be preferable to present them as pharmaceutical formulations. The formulations, both for veterinary and for human use, described herein comprise at least one active ingredient, as above defined, together with one or more acceptable carriers therefore and optionally other therapeutic ingredients. The carrier(s) must be "acceptable" in the sense of being compatible with the other ingredients of the formulation and physiologically innocuous to the recipient thereof.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present disclosure suitable for oral administration may be presented as discrete units such as, for example, capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet is made by compression or molding, optionally with one or more accessory ingredients. Compressed tablets may be prepared by compressing in a suitable machine the active ingredient in a free-flowing form such as, for example, a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom.

For administration to the eye or other external tissues e.g., mouth and skin, the formulations are preferably applied as a topical ointment or cream containing the active ingredient(s) in an amount of, for example, 0.075 to 20% w/w (including active ingredient(s) in a range between 0.1% and 20% in increments of 0.1% w/w such as, for example, 0.6% w/w, 0.7% w/w, etc.), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, *i.e.* an alcohol having two or more hydroxyl groups such as, for example, propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The oily phase of the emulsions of this disclosure may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation described herein include Tween® 60, Span® 80, cetostearyl alcohol, benzyl alcohol, myristyl alcohol, glyceryl mono-stearate and sodium lauryl sulfate.

The choice of suitable oils or fats for the formulation is based on achieving the desired cosmetic properties. The cream should preferably be a non-greasy, non-staining and washable product with suitable consistency to avoid leakage from tubes or other containers. Straight or branched chain, mono- or dibasic alkyl esters such as, for example,

di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as, for example, white soft paraffin and/or liquid paraffin or other mineral oils are used.

Pharmaceutical formulations according to the present disclosure comprise one or more compounds described herein together with one or more pharmaceutically acceptable carriers or excipients and optionally other therapeutic agents. Pharmaceutical formulations containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as, for example, calcium or sodium carbonate, lactose, lactose monohydrate, croscarmellose sodium, povidone, calcium or sodium phosphate; granulating and disintegrating agents, such as, for example, maize starch, or alginic acid; binding agents, such as, for example, cellulose, microcrystalline cellulose, starch, gelatin or acacia; and lubricating agents, such as, for example, magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as, for example, glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as, for example, peanut oil, liquid paraffin or olive oil.

Aqueous suspensions described herein contain the active materials in admixture with excipients suitable for the manufacture of aqueous suspensions. Such excipients include a suspending agent, such as, for example, sodium carboxymethylcellulose, methylcellulose, hydroxypropyl methylcelluose, sodium alginate, polyvinylpyrrolidone, gum tragacanth and gum acacia, and dispersing or wetting agents such as, for example, a naturally occurring phosphatide (*e.g.,* lecithin), a condensation product of an alkylene oxide with a fatty acid (*e.g.,* polyoxyethylene stearate), a condensation product of ethylene oxide with a long chain aliphatic alcohol (*e.g.,* heptadecaethyleneoxycetanol), a condensation product of ethylene oxide with a partial ester derived from a fatty acid and a hexitol anhydride (*e.g.,* polyoxyethylene sorbitan monooleate). The aqueous suspension may also contain one or more preservatives such as, for example, ethyl or n-propyl p-hydroxy-benzoate, one or more coloring agents, one or more flavoring agents and one or more sweetening agents, such as, for example, sucrose or saccharin.

Oil suspensions may be formulated by suspending the active ingredient in a vegetable oil, such as, for example, arachis oil, olive oil, sesame oil or coconut oil, or in a mineral oil such as, for example, liquid paraffin. The oral suspensions may contain a thickening agent, such as, for example, beeswax, hard paraffin or cetyl alcohol. Sweetening agents, such as, for example, those set forth above, and flavoring agents may be added to provide a palatable oral preparation. These compositions may be preserved by the addition of an antioxidant such as, for example, ascorbic acid.

Dispersible powders and granules described herein suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions described herein may also be in the form of oil-in-water emulsions. The oily phase may be a vegetable oil, such as, for example, olive oil or arachis oil, a mineral oil, such as, for example, liquid paraffin, or a mixture of these. Suitable emulsifying agents include naturally-occurring gums, such as, for example, gum acacia and gum tragacanth, naturally occurring phosphatides, such as, for example, soybean lecithin, esters or partial esters derived from fatty acids and hexitol anhydrides, such as, for example, sorbitan monooleate, and condensation products of these partial esters with ethylene oxide, such as, for example, polyoxyethylene sorbitan monooleate. The emulsion may also contain sweetening and flavoring agents. Syrups and elixirs may be formulated with sweetening agents, such as, for example, glycerol, sorbitol or sucrose. Such formulations may also contain a demulcent, a preservative, a flavoring or a coloring agent.

The pharmaceutical compositions described herein may be in the form of a sterile injectable preparation, such as, for example, a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as, for example, a solution in 1,3-butane-diol or prepared as a lyophilized powder.

Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as, for example, oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical composition can be prepared to provide easily measurable amounts for administration. For example, an aqueous solution intended for intravenous infusion may contain from about 3 to 500 μg of the active ingredient per milliliter of solution in order that infusion of a suitable volume at a rate of about 30 mL/hr can occur.

Formulations suitable for administration to the eye include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavored basis, usually sucrose and acacia or tragacanth; pastilles comprising the active ingredient in an inert basis such as, for example, gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for intrapulmonary or nasal administration have a particle size for example in the range of 0.1 to 500 microns (including particle sizes in a range between 0.1 and 500 microns in increments microns such as, for example, 0.5, 1, 30 microns, 35 microns, etc.), which is administered by rapid inhalation through the nasal passage or by inhalation through the mouth so as to reach the alveolar sacs. Suitable formulations include aqueous or oily solutions of the active ingredient. Formulations suitable for aerosol or dry powder administration may be prepared according to conventional methods and may be delivered with other therapeutic agents such as, for example, compounds heretofore used in the treatment or prophylaxis of conditions associated with HCV activity.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should be understood that in addition to the ingredients particularly mentioned above the formulations of this disclosure may include other agents conventional in the art having regard to the type of formulation in question, for example those suitable for oral administration may include flavoring agents.

The disclosure further provides veterinary compositions comprising at least one active ingredient as above defined together with a veterinary carrier therefore.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds described herein can also be formulated to provide controlled release of the active ingredient to allow less frequent dosing or to improve the pharmacokinetic or toxicity profile of the active ingredient. Accordingly, the disclosure also provides compositions comprising one or more compounds described herein formulated for sustained or controlled release.

Effective dose of active ingredient depends at least on the nature of the condition being treated, toxicity, whether the compound is being used prophylactically (lower doses), the method of delivery, and the pharmaceutical formulation, and will be determined by the clinician using conventional dose escalation studies.

In one embodiment, the active ingredient (i.e., one or more compounds as described herein) or pharmaceutical

composition comprising the active ingredient are effective in treating one or more of genotype 1 HCV infected subjects, genotype 2 HCV infected subjects, genotype 3 HCV infected subjects, genotype 4 HCV infected subjects, genotype 5 HCV infected subjects, and/or genotype 6 HCV infected subjects. In one embodiment, the active ingredient or pharmaceutical composition comprising the active ingredient are effective in treating genotype 1 HCV infected subjects, including genotype 1a and/or genotype 1b. In another embodiment, the active ingredient or pharmaceutical composition comprising the active ingredient are effective in treating genotype 2 HCV infected subjects, including genotype 2a, genotype 2b, genotype 2c and/or genotype 2d. In another embodiment, the active ingredient or pharmaceutical composition comprising the active ingredient are effective in treating genotype 3 HCV infected subjects, including genotype 3a, genotype 3b, genotype 3c, genotype 3d, genotype 3e and/or genotype 3f. In another embodiment, the active ingredient or pharmaceutical composition comprising the active ingredient are effective in treating genotype 4 HCV infected subjects, including genotype 4a, genotype 4b, genotype 4c, genotype 4d, genotype 4e, genotype 4f, genotype 4g, genotype 4h, genotype 4i and/or genotype 4j. In another embodiment, the active ingredient or pharmaceutical composition comprising the active ingredient are effective in treating genotype 5 HCV infected subjects, including genotype 5a. In another embodiment, the active ingredient or pharmaceutical composition comprising the active ingredient are effective in treating genotype 6 HCV infected subjects, including genotype 6a. In one embodiment, the active ingredient or pharmaceutical composition comprising the active ingredient are pangenotypic, meaning they are useful across all genotypes and drug resistant mutants thereof.

In some embodiments, the active ingredient or pharmaceutical composition comprising the active ingredient is administered, either alone or in combination with one or more therapeutic agent(s) for treating HCV (such as a HCV NS3 protease inhibitor or an inhibitor of HCV NS5B polymerase), for about 24 weeks, for about 16 weeks, or for about 12 weeks, or less. In further embodiments, the active ingredient or pharmaceutical composition comprising the active ingredient is administered, either alone or in combination with one or more therapeutic agent(s) for treating HCV (such as a HCV NS3 protease inhibitor or an inhibitor of HCV NS5B polymerase), for about 24 weeks or less, about 22 weeks or less, about 20 weeks or less, about 18 weeks or less, about 16 weeks or less, about 12 weeks or less, about 10 weeks or less, about 8 weeks or less, or about 6 weeks or less or about 4 weeks or less. The active ingredient or pharmaceutical composition comprising the active ingredient may be administered once daily, twice daily, once every other day, two times a week, three times a week, four times a week, or five times a week.

In further embodiments, a sustained virologic response is achieved at about 4 weeks, 6 weeks, 8 weeks, 12 weeks, or 16 weeks, or at about 20 weeks, or at about 24 weeks, or at about 4 months, or at about 5 months, or at about 6 months, or at about 1 year, or at about 2 years.

In some embodiments, the active ingredient or pharmaceutical composition comprising the active ingredient is administered, either alone or in combination with one or more therapeutic agent(s) for treating HCV, once daily for about 12 weeks or less to a patient infected with a hepatitis C virus of genotype 1, 2, 3, 4, 5, or 6. In some embodiments, the active ingredient or pharmaceutical composition comprising the active ingredient is administered, either alone or in combination with one or more therapeutic agent(s) for treating HCV, once daily for about 12 weeks or less to a patient infected with a hepatitis C virus of genotype 1a, 1b, 2a, 2b, 2c, 2d, 3a, 3b, 3c, 3d, 3e, 3f, 4a, 4b, 4c, 4d, 4e, 4f, 4g, 4h, 4i, 5a, or 6a.

In some embodiments, the active ingredient or pharmaceutical composition comprising the active ingredient is administered, either alone or in combination with one or more therapeutic agent(s) for treating HCV, once daily for about 8 weeks or less to a patient infected with a hepatitis C virus of genotype 1, 2, 3, 4, 5, or 6. In some embodiments, the active ingredient or pharmaceutical composition comprising the active ingredient is administered, either alone or in combination with one or more therapeutic agent(s) for treating HCV, once daily for about 8 weeks or less to a patient infected with a hepatitis C virus of genotype 1a, 1b, 2a, 2b, 2c, 2d, 3a, 3b, 3c, 3d, 3e, 3f, 4a, 4b, 4c, 4d, 4e, 4f, 4g, 4h, 4i, 5a, or 6a.

In some embodiments, the active ingredient or pharmaceutical composition comprising the active ingredient is administered, either alone or in combination with one or more therapeutic agent(s) for treating HCV, once daily for about 6 weeks or less to a patient infected with a hepatitis C virus of genotype 1, 2, 3, 4, 5, or 6. In some embodiments, the active ingredient or pharmaceutical composition comprising the active ingredient is administered, either alone or in combination with one or more therapeutic agent(s) for treating HCV, once daily for about 6 weeks or less to a patient infected with a hepatitis C virus of genotype 1a, 1b, 2a, 2b, 2c, 2d, 3a, 3b, 3c, 3d, 3e, 3f, 4a, 4b, 4c, 4d, 4e, 4f, 4g, 4h, 4i, 5a, or 6a.

**Routes of Administration**

One or more compounds described herein (herein referred to as the active ingredients) are administered by any route appropriate to the condition to be treated. Suitable routes include oral, rectal, nasal, topical (including buccal and sublingual), vaginal and parenteral (including subcutaneous, intramuscular, intravenous, intradermal, intrathecal and epidural), and the like. It will be appreciated that the preferred route may vary with for example the condition of the

recipient. An advantage of the compounds of this disclosure is that they are orally bioavailable and can be dosed orally.

**HCV Combination Therapy**

In another embodiment, non-limiting examples of suitable combinations include combinations of one or more compounds of formula (I), (II), and (A1-A6) with one or more interferons, ribavirin or its analogs, HCV NS3 protease inhibitors, alpha-glucosidase 1 inhibitors, hepatoprotectants, nucleoside or nucleotide inhibitors of HCV NS5B polymerase, non-nucleoside inhibitors of HCV NS5B polymerase, HCV NS5A inhibitors, TLR-7 agonists, cyclophillin inhibitors, HCV IRES inhibitors, pharmacokinetic enhancers, and other drugs or therapeutic agents for treating HCV.

More specifically, one or more compounds of the present as described herein may be combined with one or more compounds selected from the group consisting of

1) interferons, *e.g.,* pegylated rIFN-alpha 2b (PEG-Intron®), pegylated rIFN-alpha 2a (Pegasys®), rIFN-alpha 2b (Intron® A), rIFN-alpha 2a (Roferon®-A), interferon alpha (MOR-22, OPC-18, Alfaferone®, Alfanative®, Multiferon®, subalin), interferon alfacon-1 (Infergen®), interferon alpha-nl (Wellferon), interferon alpha-n3 (Alferon®), interferon-beta (Avonex®, DL-8234), interferon-omega (omega DUROS®, Biomed® 510), albinterferon alpha-2b (Albuferon®), IFN alpha-2b XL, BLX-883 (Locteron®), DA-3021, glycosylated interferon alpha-2b (AVI-005), PEG-Infergen, PEGylated interferon lambda-1 (PEGylated IL-29), and belerofon®;

2) ribavirin and its analogs, *e.g.,* ribavirin (Rebetol®, Copegus®), and taribavirin (Viramidine®);

3) HCV NS3 protease inhibitors, e.g., boceprevir (SCH-503034, SCH-7), telaprevir (VX-950), TMC435350, BI-1335, BI-1230, MK-7009, VBY-376, VX-500, GS-9256, GS-9451, BMS-605339, PHX-1766, AS-101, YH-5258, YH5530, YH5531, ABT-450, ACH-1625, ITMN-191, AT26893, MK5172, MK6325, and MK2748;

4) alpha-glucosidase 1 inhibitors, *e.g.,* celgosivir (MX-3253), Miglitol, and UT-231B;

5) hepatoprotectants, *e.g.,* emericasan (IDN-6556), ME-3738, GS-9450 (LB-84451), silibilin, and MitoQ;

6) nucleoside or nucleotide inhibitors of HCV NS5B polymerase, *e.g.,* R1626, R7128 (R4048), IDX184, IDX-102, BCX-4678, valopicitabine (NM-283), MK-0608, sofosbuvir (GS-7977 (formerly PSI-7977)), VLX-135 (formerly ALS-2200), and INX-189 (now BMS986094);

7) non-nucleoside inhibitors of HCV NS5B polymerase, *e.g.,* PF-868554, VCH-759, VCH-916, JTK-652, MK-3281, GS-9190, VBY-708, VCH-222, A848837, ANA-598, GL60667, GL59728, A-63890, A-48773, A-48547, BC-2329, VCH-796 (nesbuvir), GSK625433, BILN-1941, XTL-2125, ABT-072, ABT-333, GS-9669, PSI-7792, and GS-9190;

8) HCV NS5A inhibitors, *e.g.,* AZD-2836 (A-831), BMS-790052, ACH-3102, ACH-2928, MK8325, MK4882, MK8742, PSI-461, IDX719, GS-5885, and A-689;

9) TLR-7 agonists, *e.g.,* imiquimod, 852A, GS-9524, ANA-773, ANA-975 (isatoribine), AZD-8848 (DSP-3025), and SM-360320;

10) cyclophillin inhibitors, *e.g.,* DEBIO-025, SCY-635, and NIM811;

11) HCV IRES inhibitors, *e.g.,* MCI-067;

12) pharmacokinetic enhancers, *e.g.,* BAS-100, SPI-452, PF-4194477, TMC-41629, GS-9350 (cobicistat), GS-9585, and roxythromycin; and

13) other drugs for treating HCV, *e.g.,* thymosin alpha 1 (Zadaxin), nitazoxanide (Alinea, NTZ), BIVN-401 (virostat), PYN-17 (altirex), KPE02003002, actilon (CPG-10101), GS-9525, KRN-7000, civacir, GI-5005, XTL-6865, BIT225, PTX-111, ITX2865, TT-033i, ANA 971, NOV-205, tarvacin, EHC-18, VGX-410C, EMZ-702, AVI 4065, BMS-650032, BMS-791325, Bavituximab, MDX-1106 (ONO-4538), Oglufanide, and VX-497 (merimepodib).

In yet another embodiment, the present application discloses pharmaceutical compositions comprising a compound as described herein, or a pharmaceutically acceptable salt, solvate, and/or ester thereof, in combination with at least one additional therapeutic agent, and a pharmaceutically acceptable carrier or excipient.

More specifically, the additional therapeutic agent may be combined with one or more compounds selected from the group consisting of non-nucleoside inhibitors of HCV NS5B polymerase (ABT-072 and ABT-333), HCV NS5A inhibitors (ACH-3102 and ACH-2928) and HCV NS3 protease inhibitors (ABT-450 and ACH-125).

In another embodiment, the therapeutic agent used in combination with the pharmaceutical compositions as described herein can be any agent having a therapeutic effect when used in combination with the pharmaceutical compositions as described herein. For example, the therapeutic agent used in combination with the pharmaceutical compositions as described herein can be interferons, ribavirin analogs, NS3 protease inhibitors, NS5B polymerase inhibitors, alpha-glucosidase 1 inhibitors, hepatoprotectants, non-nucleoside inhibitors of HCV, and other drugs for treating HCV.

In another embodiment, the additional therapeutic agent used in combination with the pharmaceutical compositions as described herein is a cyclophillin inhibitor, including for example, a cyclophilin inhibitor disclosed in WO/2013/185093. Non-limiting examples include one or more compounds selected from the group consisiting of:

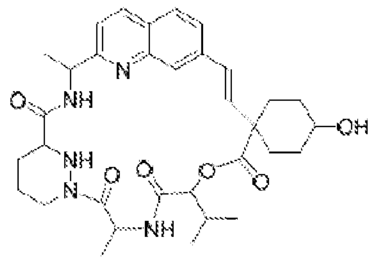,
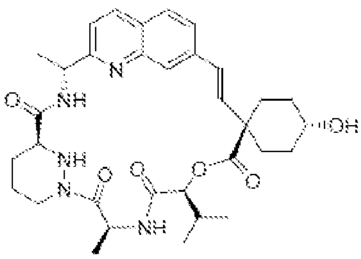,
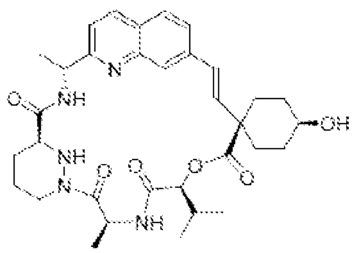,

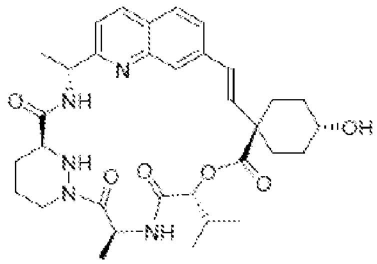,
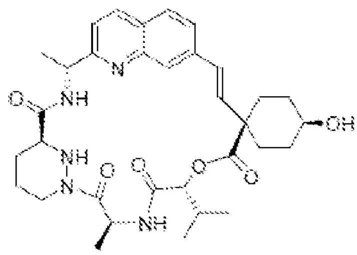,
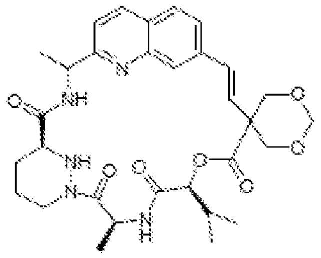,

and

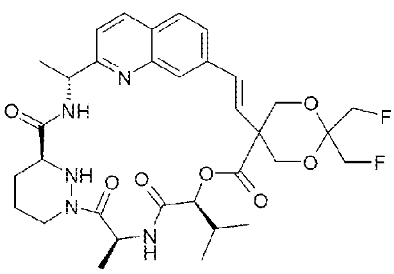,

and stereoisomers and mixtures of stereoisomers thereof.

In another embodiment, the additional therapeutic agent used in combination with the pharmaceutical compositions as described herein is a non-nucleoside inhibitor of HCV NS5B polymerase. A non-limiting example includes GS-9669.

Examples of additional anti-HCV agents which can be combined with the compositions provided herein include, without limitation, the following:

A. interferons, for example, pegylated rIFN-alpha 2b (PEG-Intron), pegylated rIFN-alpha 2a (Pegasys), rIFN-alpha 2b (Intron A), rIFN-alpha 2a (Roferon-A), interferon alpha (MOR-22, OPC-18, Alfaferone, Alfanative, Multiferon, subalin), interferon alfacon-1 (Infergen), interferon alpha-nl (Wellferon), interferon alpha-n3 (Alferon), interferon-beta (Avonex, DL-8234), interferon-omega (omega DUROS, Biomed 510), albinterferon alpha-2b (Albuferon), IFN alpha XL, BLX-883 (Locteron), DA-3021, glycosylated interferon alpha-2b (AVI-005), PEG-Infergen, PEGylated interferon lambda (PEGylated IL-29), or belerofon, IFN alpha-2b XL, rIFN-alpha 2a, consensus IFN alpha, infergen,

rebif, pegylated IFN-beta, oral interferon alpha, feron, reaferon, intermax alpha, r-IFN-beta, and infergen + actimmuneribavirin and ribavirin analogs, e.g., rebetol, copegus, VX-497, and viramidine (taribavirin);

B. NS5A inhibitors, for example, Compound B (described below), Compound C (described below), ABT-267, Compound D (described below), JNJ-47910382, daclatasvir (BMS-790052), ABT-267, MK-8742, EDP-239, IDX-719, PPI-668, GSK-2336805, ACH-3102, A-831, A-689, AZD-2836 (A-831), AZD-7295 (A-689), and BMS-790052;

C. NS5B polymerase inhibitors, for example, Compound E (described below), Compound F (described below), ABT-333, Compound G (described below), ABT-072, Compound H (described below), tegobuvir (GS-9190), GS-9669, TMC647055, setrobuvir (ANA-598), filibuvir (PF-868554), VX-222, IDX-375, IDX-184, IDX-102, BI-207127, valopicitabine (NM-283), PSI-6130 (R1656), PSI-7851, BCX-4678, nesbuvir (HCV-796), BILB 1941, MK-0608, NM-107, R7128, VCH-759, GSK625433, XTL-2125, VCH-916, JTK-652, MK-3281, VBY-708, A848837, GL59728, A-63890, A-48773, A-48547, BC-2329, BMS-791325, and BILB-1941;

D. NS3 protease inhibitors, for example, Compound I, Compound J, Compound K, ABT-450, Compound L (described below), simeprevir (TMC-435), boceprevir (SCH-503034), narlaprevir (SCH-900518), vaniprevir (MK-7009), MK-5172, danoprevir (ITMN-191), sovaprevir (ACH-1625), neceprevir (ACH-2684), Telaprevir (VX-950), VX-813, VX-500, faldaprevir (BI-201335), asunaprevir (BMS-650032), BMS-605339, VBY-376, PHX-1766, YH5531, BILN-2065, and BILN-2061;

E. alpha-glucosidase 1 inhibitors, for example, celgosivir (MX-3253), Miglitol, and UT-231B;

F. hepatoprotectants, e.g., IDN-6556, ME 3738, MitoQ, and LB-84451;

G. non-nucleoside inhibitors of HCV, e.g., benzimidazole derivatives, benzo-1,2,4-thiadiazine derivatives, and phenylalanine derivatives; and

H. other anti-HCV agents, e.g., zadaxin, nitazoxanide (alinea), BIVN-401 (virostat), DEBIO-025, VGX-410C, EMZ-702, AVI 4065, bavituximab, oglufanide, PYN-17, KPE02003002, actilon (CPG-10101), KRN-7000, civacir, GI-5005, ANA-975, XTL-6865, ANA 971, NOV-205, tarvacin, EHC-18, and NIM811.

Compound B is an NS5A inhibitor and is represented by the following chemical structure:

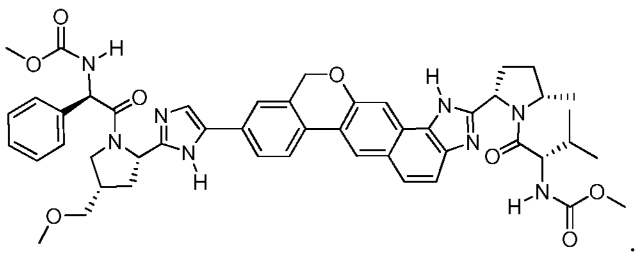

Compound C ("ledipasvir") is an NS5A inhibitor and is represented by the following chemical structure:

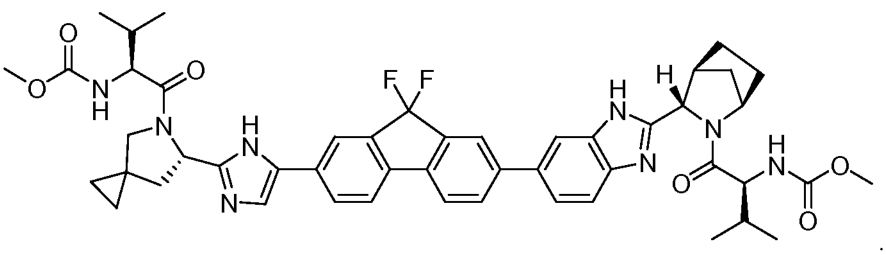

Compound D is an NS5A inhibitor and is represented by the following chemical structure:

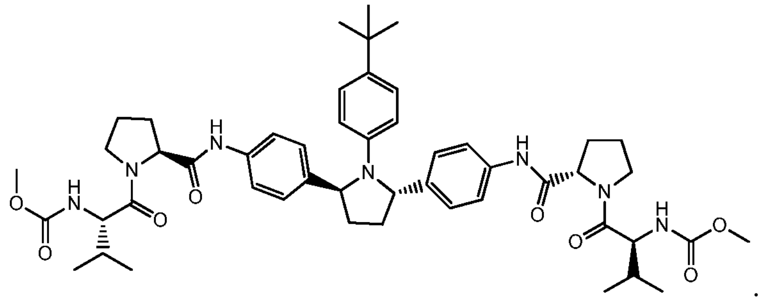

See U.S. Publication No. 2013/0102525 and references therein.

Compound E is an NS5B Thumb II polymerase inhibitor and is represented by the following chemical structure:

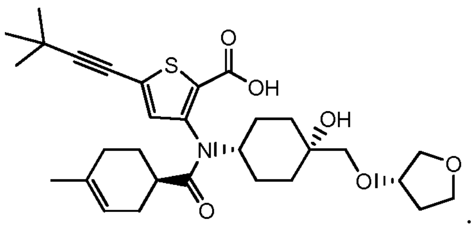

Compound F is a nucleotide inhibitor prodrug designed to inhibit replication of viral RNA by the HCV NS5B polymerase, and is represented by the following chemical structure:

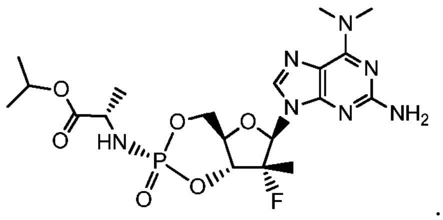

Compound G is an HCV polymerase inhibitor and is represented by the following structure:

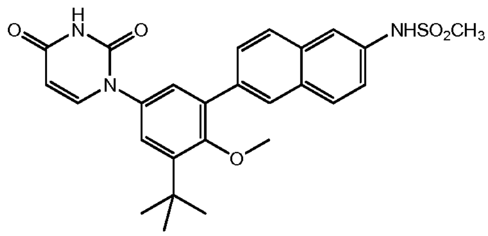

See U.S. Publication No. 2013/0102525 and references therein.

Compound H is an HCV polymerase inhibitor and is represented by the following structure:

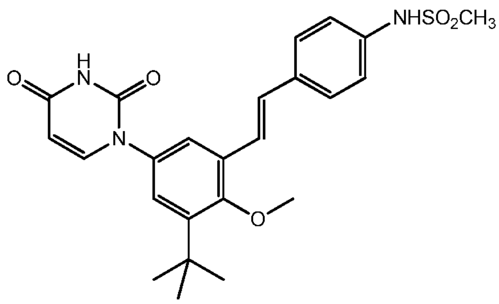

See U.S. Publication No. 2013/0102525 and references therein.

Compound I is an HCV protease inhibitor and is represented by the following chemical structure:

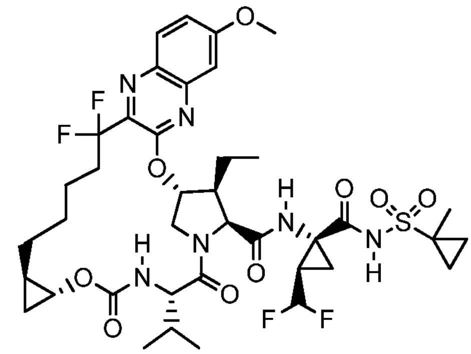

.

See WO 2014/008285, filed July 2, 2013, and references therein.

Compound J is an HCV protease inhibitor and is represented by the following chemical structure:

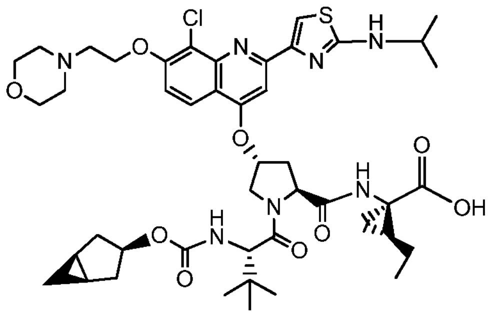

.

Compound K is an HCV protease inhibitor and is represented by the following chemical structure:

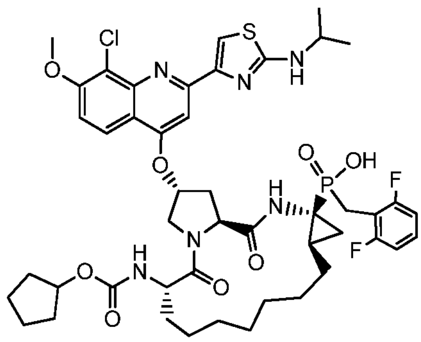

.

Compound L is an HCV protease inhibitor and is represented by the following chemical structure:

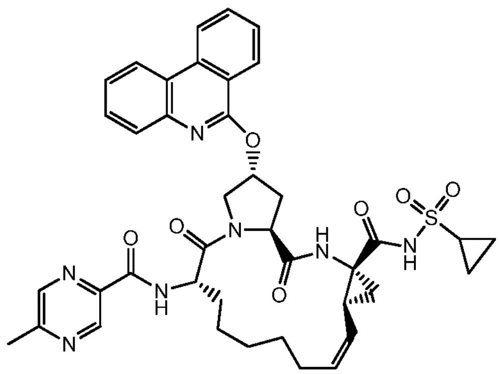

.

See U.S. Publication No. 2013/0102525 and references therein.

In one embodiment, the additional therapeutic agent used in combination with the pharmaceutical compositions as described herein is a HCV NS3 protease inhibitor. Non-limiting examples include one or more compounds selected from the group consisting of:

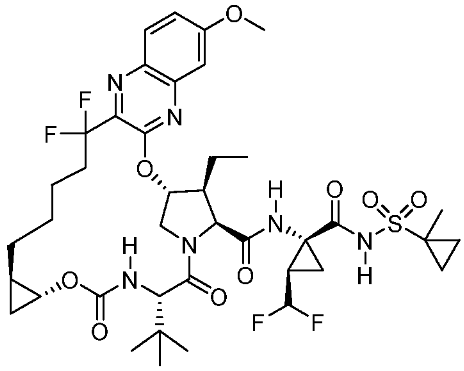

,

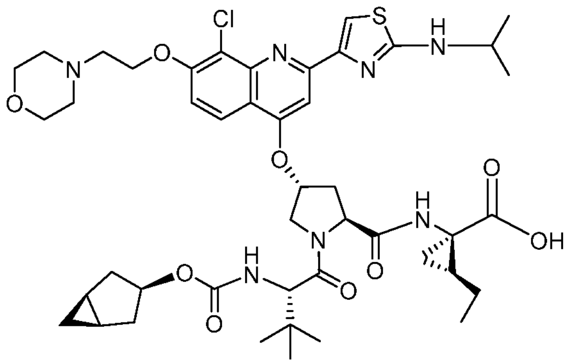

,

and

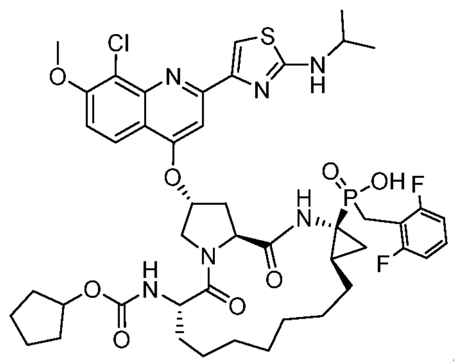

.

In another embodiment, the present application provides for a method of treating hepatitis C in a human patient in need thereof comprising administering to the patient a therapeutically effective amount of a pharmaceutical composition as described herein and an additional therapeutic selected from the group consisting of pegylated rIFN-alpha 2b, pegylated rIFN-alpha 2a, rIFN-alpha 2b, IFN alpha-2b XL, rIFN-alpha 2a, consensus IFN alpha, infergen, rebif, locteron, AVI-005, PEG-infergen, pegylated IFN-beta, oral interferon alpha, feron, reaferon, intermax alpha, r-IFN-beta, infergen + actimmune, IFN-omega with DUROS, albuferon, rebetol, copegus, levovirin, VX-497, viramidine (taribavirin), A-831, A-689, NM-283, valopicitabine, R1626, PSI-6130 (R1656), HCV-796, BILB 1941, MK-0608, NM-107, R7128, VCH-759, PF-868554, GSK625433, XTL-2125, SCH-503034 (SCH-7), VX-950 (Telaprevir), ITMN-191, and BILN-2065, MX-3253 (celgosivir), UT-231B, IDN-6556, ME 3738, MitoQ, and LB-84451, benzimidazole derivatives, benzo-1,2,4-thiadiazine derivatives, and phenylalanine derivatives, zadaxin, nitazoxanide (alinea), BIVN-401 (virostat), DEBIO-025, VGX-410C,

EMZ-702, AVI 4065, bavituximab, oglufanide, PYN-17, KPE02003002, actilon (CPG-10101), KRN-7000, civacir, GI-5005, ANA-975 (isatoribine), XTL-6865, ANA 971, NOV-205, tarvacin, EHC-18, and NIM811 and a pharmaceutically acceptable carrier or excipient.

In another embodiment is provided a pharmaceutical composition comprising a compound of formula (I) as described herein and sofosbuvir and/or Compound C and/or Compound B and optionally an interferon or ribavirin.

It is contemplated that additional therapeutic agents will be administered in a manner that is known in the art and the dosage may be selected by someone of skill in the art. For example, additional therapeutic agents may be administered in a dose from about 0.01 milligrams to about 2 grams per day.

In one embodiment, the disclosure provides a compound of formula:

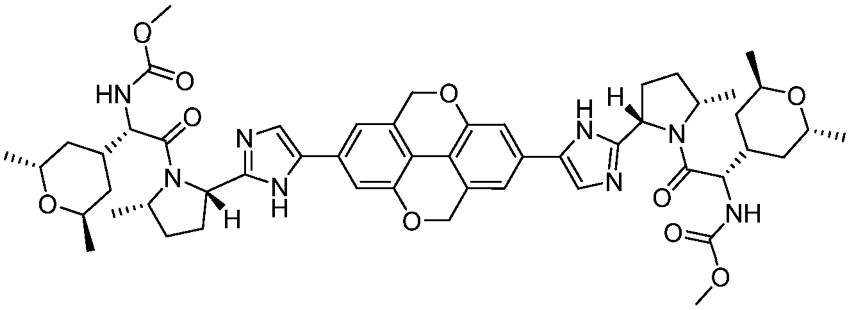

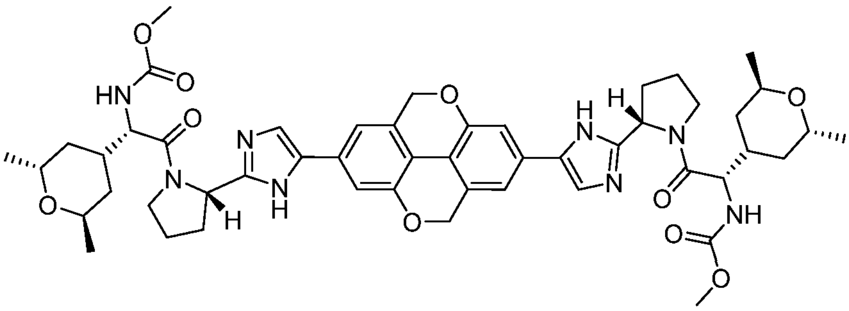

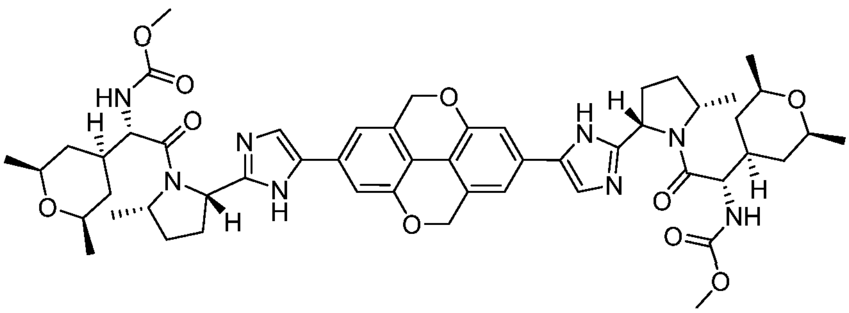

or a pharmaceutically acceptable salt thereof.

The disclosure will now be illustrated by the following Examples. The following abbreviations are used throughout the specification, including the Examples.

| | |
|---|---|
| %F | % Bioavailability |
| °C | Degree Celsius |
| Ac | Acetate |
| apprx. | Approximate |
| AUC | Area under the curve |
| Bn | Benzyl |
| BOC/Boc | tert-Butoxycarbonyl |

(continued)

| | |
|---|---|
| %F | % Bioavailability |
| br | Broad |
| calc'd | Calculated |
| $CC_{50}$ | 50% Cytotoxicity concentration |
| d | Doublet |
| DCM | Dichloromethane |
| dd | Doublet of doublets |
| DIPEA | N,N-Diisopropylethylamine |
| DMEM | Eagle's minimal essential medium |
| DMF | Dimethylformamide |
| DMSO | Dimethylsulfoxide |
| EA | Ethyl acetate |
| $EC_{50}$ | Half maximal effective concentration |
| EDTA | Ethylenediaminetetraacetic acid |
| ESI | Electrospray ionization |
| Et | Ethyl |
| FBS | Fetal bovine serum |
| g | Gram |
| HATU | 2-(1H-7-Azabenzotriazol-1-yl)-1,1,3 ,3-tetramethyl uronium hexafluorophosphate Methanaminium |
| HPLC | High performance liquid chromatography |
| hr/h | Hour |
| Hz | Hertz |
| i.d. | Inner diameter |
| IPAm | Isopropylamine |
| IV | Intravenous |
| J | Coupling constant |
| L | Liter |
| LC | Liquid chromatography |
| LCMS | Liquid chromatography mass spectrometry |
| M | Molar |
| m | Multiplet |
| m/z | Mass to charge |
| M+ | Mass peak |
| Me | Methyl |
| mg | Milligram |
| MHz | Megahertz |
| min | Minute |
| mL | Milliliter |
| mM | Millimolar |

(continued)

| | |
|---|---|
| %F | % Bioavailability |
| mm | Millimeter |
| mmol | Millimole |
| MS | Mass spectrometry |
| N | Normal |
| NADPH | Nicotinamide adenine dinucleotide phosphate |
| nm | Nanometer |
| NMR | Nuclear magnetic resonance |
| o/n | Over night |
| Papp | Apparent permeability |
| PE | Petroleum ether |
| Ph | Phenyl |
| PBS | Phosphate buffer system |
| Pd/C | Palladium on carbon |
| PEG | Polyethylene glycol |
| Pt/C | Platinum on carbon |
| q | Quartet |
| quant | Quantitative |
| rt/RT | Room temperature |
| s | Singlet |
| SFC | Supercritical fluid chromatography |
| S-Phos | 2-Dicyclohexylphosphino-2',6'-dimethoxybiphenyl |
| t | Triplet |
| t-Bu | tert-Butyl |
| TEA | Triethylamine |
| Tf | Trifluoromethanesulfonate |
| TFA | Trifluoroacetic acid |
| THF | Tetrahydrofuran |
| TLC | Thin layer chromatography |
| UV | Ultraviolet |
| w/w | Weight to weight |
| $\delta$ | Chemical shift |
| $\mu$L | Microliter |
| $\mu$m | Micrometer |
| $\mu$M | Micromolar |

## EXAMPLES

### Example 1

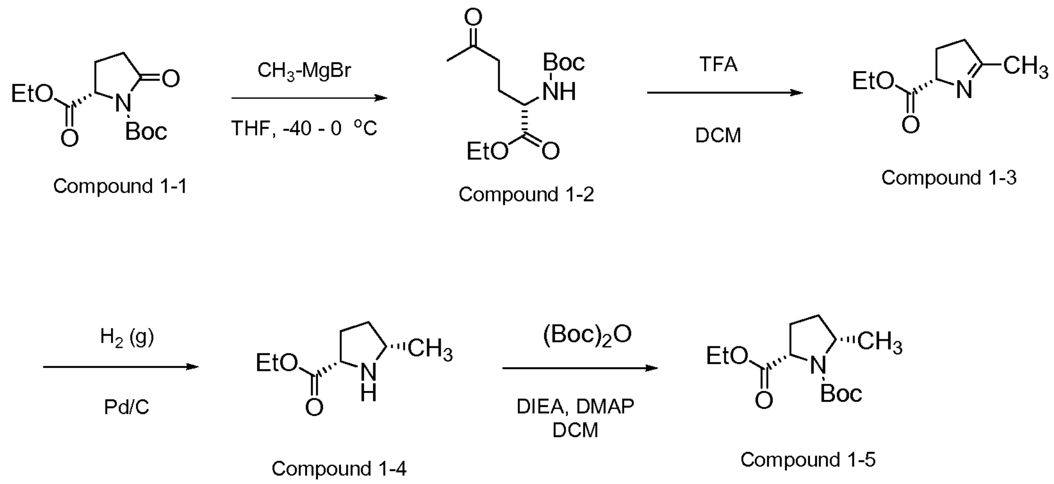


Compound 1-1 Compound 1-2 Compound 1-3

Compound 1-4 Compound 1-5

**(S)-Ethyl 2-(tert-butoxycarbonylamino)-5-oxohexanoate (Compound 1-2).**

A solution of ethyl *N*-Boc (S)-pyroglutamate (20.0 g, 77.7 mmol) (Compound 1-1) was in anhydrous THF (150 mL) in a two neck round bottom under argon was cooled to -40 °C. Methyl-magnesium bromide solution (3.0 M in ether, 28.5 mL, 85.5 mmol) was added to the reaction mixture dropwise over 30 minutes. The reaction was stirred for 4 hrs at -40 °C then for 1 hr at 0 °C. The reaction was partitioned between ethyl acetate and saturated ammonium chloride solution and acidified with 1 N HCl. The aqueous layer was extracted two more times with ethylacetate. The organic layers were combined and dried with sodium sulfate. The crude material was purified by column chromatography (20% - 40% EtOAc/hexanes) to yield (S)-ethyl 2-(tert-butoxycarbonylamino)-5-oxohexanoate as a viscous oil and was used directly in the following step.

**(S)-Ethyl 5-methyl-3,4-dihydro-2H-pyrrole-2-carboxylate (Compound 1-3).**

(S)-ethyl 2-(tert-butoxycarbonylamino)-5-oxohexanoate in a 1 L flask was treated with a trifluoro acetic acid / dichloromethane solution (1:1 mixture, 100 mL). Effervescence was observed and the mixture was allowed to stir for 4 hours at room temperature. After which time the volatiles were removed *in vacuo* to yield (S)-ethyl 5-methyl-3,4-dihydro-2H-pyrrole-2-carboxylate as an oil, and used directly in the following step.

**(2S,5S)-Ethyl 5-methylpyrrolidine-2-carboxylate (Compound 1-4).**

The crude imine 1-3 in a 1 L flask was dissolved with ethanol (400 mL) was evacuated and charged with argon three times (3x). Palladium on carbon (apprx. 750 mg, 10% w/w, dry) was added and the reaction was evacuated of gas and charged with hydrogen gas (3x). The reaction was allowed to stir under atmospheric hydrogen for 16 hours. The mixture was filtered through a plug of celite and the filtrate was concentrated *in vacuo.* Diethyl ether was added to the oil and a precipitate formed. The mixture was filtered to yield (2S,5S)-ethyl 5-methylpyrrolidine-2-carboxylate, as a white solid (10.6 g, 67.4 mmol, 86.7% over three steps). $^1H$ NMR (400 MHz, $CDCl_3$) $\delta$ 4.48 (dd, 1H), 4.27 (q, 2H), 3.92 - 3.80 (m, 1H), 2.52 - 2.36 (m, 1H), 2.32 - 2.13 (m, 2H), 1.75 - 1.60 (m, 1H), 1.51 (d, 3H), 1.30 (t, 3H).

**(2S,5S)-1-Tert-butyl 2-ethyl 5-methylpyrrolidine-1,2-dicarboxylate (Compound 1-5).**

To a solution of (2S,5S)-ethyl 5-methylpyrrolidine-2-carboxylate (7.0 g, 44.5 mmol) in dichloromethane (250 mL), ditertbutylanhydride (10.7 g, 49.0 mmol), diisopropylethylamine (17.1 mL, 98.0 mmol) dropwise over 10 minutes, and dimethyl amino pyridine (0.27 g, 2.23 mmol) were added successively. Effervescence was observed and the mixture was allowed to stir for 16 hours at room temperature. The reaction was washed with HCl (250 mL of 1 N). The organic layer was then dried with sodium sulfate. The crude material was purified by column chromatography (5% - 25%

EtOAc/hexanes) to yield (2S,5S)-1-tert-butyl2-ethyl 5-methylpyrrolidine-1,2-dicarboxylate as an oil (6.46 g, 25.1 mmol, 56%). LCMS-ESI$^+$: calc'd for $C_{13}H_{23}NO_4$: 257.16 ($M^+$); Found: 258.70 ($M+H^+$).

**(2S,5S)-1-(Tert-butoxycarbonyl)-5-methylpyrrolidine-2-carboxylic acid (Compound 1-6).**

To a solution of (2S,5S)-1-tert-butyl2-ethyl 5-methylpyrrolidine-1,2-dicarboxylate (6.46 g, 25.1 mmol) in ethanol (20 mL) was added lithium hydroxide monohydrate (2.11 g, 50.2 mmol) and deionized water (12 mL). The mixture was allowed to stir for 16 hours then partitioned between ethylacetate and a 1:1 mixture of saturated brine and 1 N HCl. The aqueous layer was extracted an additional time with ethyl acetate. The organic layers were combined, dried with sodium sulfate and the solvent was removed *in vacuo* to yield (2S,5S)-1-(tert-butoxycarbonyl)-5-methylpyrrolidine-2-carboxylic acid as a white solid (quant.) and was used directly in the following step.

**Reference Example 2**

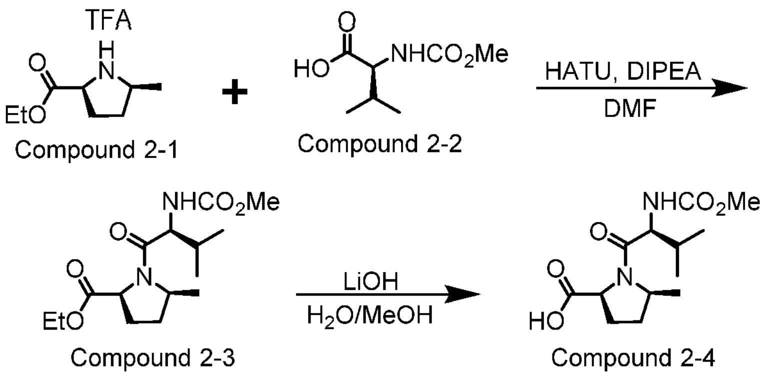

Compound 2-1 Compound 2-2 Compound 2-3 Compound 2-4

**(2S,5S)-Ethyl 1-((S)-2-(methoxycarbonylamino)-3-methylbutanoyl)-5-methylpyrrolidine-2-carboxylate (Compound 2-3).**

(2S,5S)-Ethyl 5-methylpyrrolidine-2-carboxylate-TFA (10.0 g, 39.3 mmol) (Compound 2-1), (S)-2-(methoxycarbonylamino)-3-methylbutanoic acid (6.88 g, 39.3 mmol) (Compound 2-2) and HATU (14.9 g, 39.3 mmol) were combined in DMF (100 mL) and DIPEA (15.0 mL, 86.5 mmol) was added. After stirring for 1 h at RT, the reaction mixture was diluted with EtOAc. The organic phase was washed successively with 10% HCl, saturated aqueous $NaHCO_3$ and brine, then dried over $MgSO_4$, filtered and concentrated under reduced pressure to afford (2S,5S)-ethyl 1-((S)-2-(methoxycarbonylamino)-3-methylbutanoyl)-5-methylpyrrolidine-2-carboxylate. The crude material was carried on without further purification.

**(2S,5S)-1-((S)-2-(Methoxycarbonylamino)-3-methylbutanoyl)-5-methylpyrrolidine-2-carboxylic acid (Compound 2-4).**

(2S,5S)-Ethyl 1-((S)-2-(methoxycarbonylamino)-3-methylbutanoyl)-5-methylpyrrolidine-2-carboxylate (39.3 mmol, assuming complete conversion from the previous transformation) was suspended in MeOH (200 mL) and aqueous LiOH (1.0 M, 100 mL, 100 mmol) was added. The reaction mixture was stirred o/n, then concentrated under reduced pressure to remove most of the MeOH. The aqueous solution was washed 2x with DCM before being acidified to pH~1-2 with 10% HCl. The acidic aqueous phase was then extracted 5x with EtOAc. The combined EtOAc extracts were dried over $MgSO_4$ filtered and concentrated under reduced pressure to afford (2S,5S)-1-((S)-2-(Methoxycarbonylamino)-3-methylbutanoyl)-5-methylpyrrolidine-2-carboxylic acid (6.89 g, 56% over 2 steps).

**Example 13**

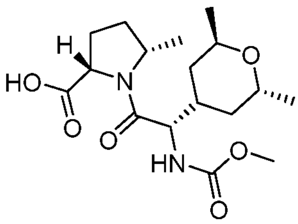

**(2S,5S)-1-((S)-2-((2R,6R)-2,6-dimethyltetrahydro-2H-pyran-4-yl)-2-(methoxycarbonylamino)acetyl)-5-methylpyrrolidine-2-carboxylic acid (Compound 13)**

(2S,5S)-1-((S)-2-((2R,6R)-2,6-dimethyltetrahydro-2H-pyran-4-yl)-2-(methoxycarbonylamino)acetyl)-5-methylpyrrolidine-2-carboxylic acid was synthesized according to the method described in Example 2 substituting (S)-2-(methoxycarbonylamino)-3-methylbutanoic acid with (S)-2-((2R,6R)-2,6-dimethyltetrahydro-2H-pyran-4-yl)-2-(methoxycarbonylamino)acetic acid. $^1$H NMR (400 MHz, Chloroform-d) δ 5.33 - 5.16 (m, 1H), 4.70 - 4.59 (m, 1H), 4.54 (t, 1H), 4.34 - 4.19 (m, 2H), 4.12 (q, 1H), 3.78 - 3.70 (m, 1H), 3.67 (s, 3H), 2.37 - 2.17 (m, 3H), 2.15 - 2.07 (m, 1H), 2.04 (s, 1H), 1.84 - 1.73 (m, 1H), 1.82 - 1.43 (m, 3H), 1.32 (d, 3H), 1.26 (d, 4H), 1.11 (d, 3H), 0.96 (q, 1H). LCMS-ESI+ calc'd for $C_{17}H_{29}N_2O_6$: 357.19 ; Found: 357.08.

**Example 14**

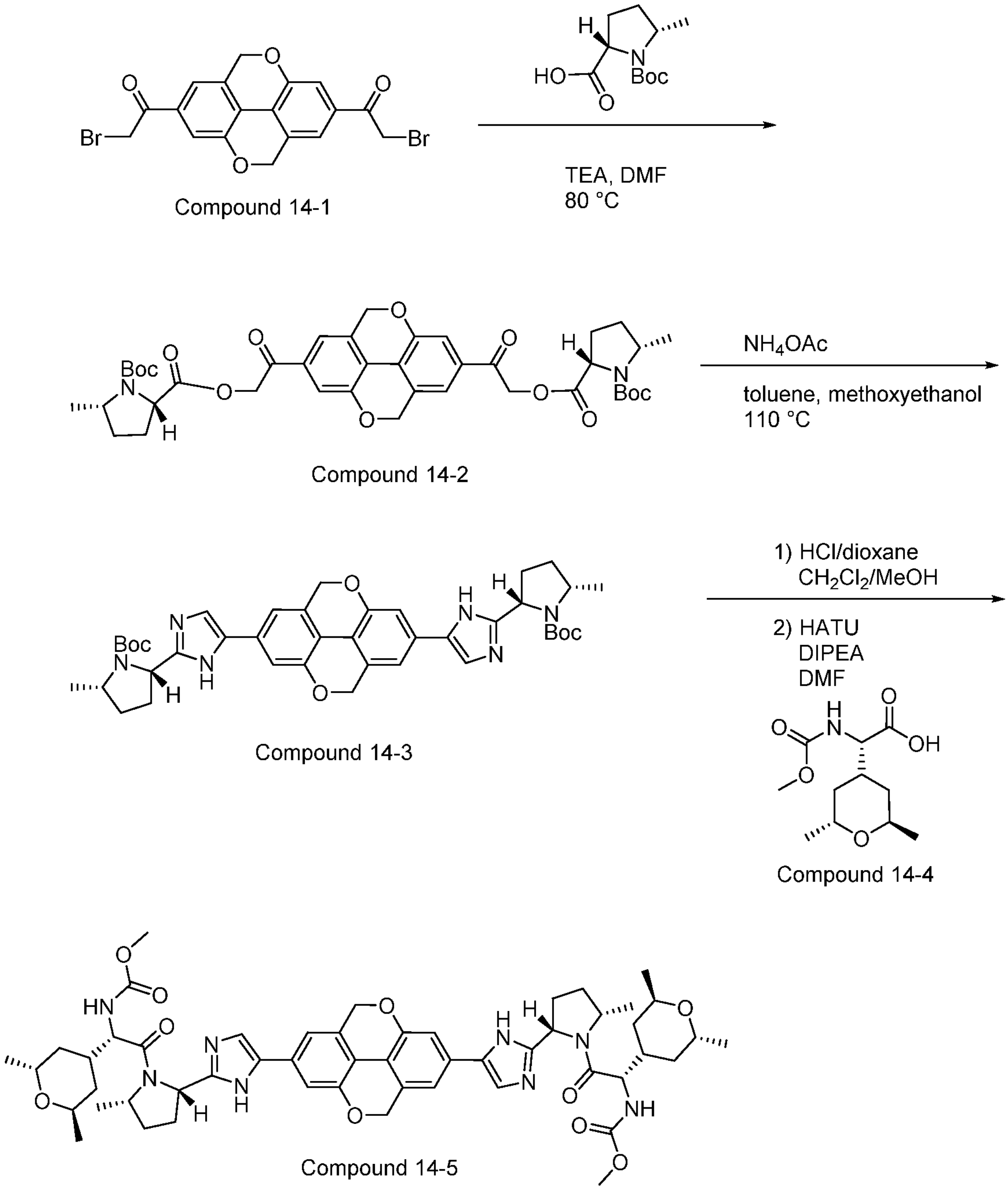


Compound 14-1

Compound 14-2

Compound 14-3

Compound 14-4

Compound 14-5

**2S,2 'R,5S,5'S)-1-tert-butyl'2,2-2,2 '-(5,10-dihydrochromeno[5,4,3-cde] chromene-2,7-diyl)bis(2-oxoethane-2,1-diyl)bis(5-methylpyrrolidine-1,2-dicarboxylate) (Compound 14-2).**

To a mixture of 1,1'-(5,10-dihydrochromeno[5,4,3-cde]chromene-2,7-diyl)bis(2-bromoethanone) (1.6 g, 3.5 mmol) (Compound 14-1) and (2S,5S)-1-(tert-butoxycarbonyl)-5-methylpyrrolidine-2-carboxylic acid (2.0 g, 8.8 mmol) in dimethylformamide (19.6 mL) was added triethylamine (1.2 mL, 8.8 mmol). The solution was heated to 80 °C in an external oil bath for 4 hours. Upon completion, the reaction mixture was added slowly to a flask of rapidly stirring water. The precipitate that formed was filtered through a nylon membrane frit and dried in vacuo to give (2S,2'R,5S,5'S)-1-tert-butyl '2,2-2,2'-(5,10-dihydrochromeno[5,4,3-cde]chromene-2,7-diyl)bis(2-oxoethane-2,1-diyl) bis(5-methylpyrrolidine-1,2-dicarboxylate) (2.44 g, 92%) as a white solid. $^1H$ NMR (400 MHz, Chloroform-d) $\delta$ 7.38 (s, 2H), 7.33 (s, 2H), 5.56 - 5.37 (m, 1H), 5.31 (s, 4H), 5.26 - 5.14 (m, 1H), 4.45 (d, *J* = 33.3 Hz, 2H), 4.00 (d, *J* = 45.2 Hz, 2H), 2.59 (s, 0H), 2.30 (d, *J* = 7.8 Hz, 4H), 2.16 - 2.00 (m, 2H), 1.81 - 1.54 (m, 3H), 1.45 (d, *J* = 12.5 Hz, 18H), 1.31 (s, 6H).

**(2S,2'S,5S,5'S)-tert-butyl 5,5'-(5,5'-(5,10-dihydrochromeno[5,4,3-cde]chromene-2,7-diyl)bis(1H-imidazole-5,2-diyl))bis(2-methylpyrrolidine-1-carboxylate) (Compound 14-3).**

A mixture of (2S,2'R,5S,5'S)-1-tert-butyl'2,2-2,2'-(5,10-dihydrochromeno[5,4,3-cde]chromene-2,7-diyl)bis(2-oxoethane-2,1-diyl)bis(5-methylpyrrolidine-1,2-dicarboxylate) (2.44 g, 3.25 mmol) and ammonium acetate (5.02 g, 65.169 mmol) was suspended in toluene (32 mL) and methoxyethanol (3.2 mL). The reaction mixture was heated to 110 °C for 18 hours, and then cooled to room temperature. The residue was purified by flash column chromatography (silica, 0-8% Methanol in DCM) to give (2S,2'S,5S,5'S)-tert-butyl 5,5'-(5,5'-(5,10-dihydrochromeno[5,4,3-cde]chromene-2,7-diyl)bis(1H-imidazole-5,2-diyl))bis(2-methylpyrrolidine-1-carboxylate) (2.03 g, 88%). LCMS-ESI+: calc'd for $C_{40}H_{49}N_6O_6$: 709.36 (M+); Found: 709.59 (M+H+). $^1H$ NMR (400 MHz, Chloroform-*d*) $\delta$ 7.21 - 6.91 (m, 4H), 5.24 (s, 4H), 5.02 - 4.87 (m, 2H), 3.96 (s, 2H), 3.76 - 3.47 (m, 1H), 2.86 (s, 2H), 2.08 (d, *J* = 1.9 Hz, 6H), 1.88 (d, *J* = 24.9 Hz, 1H), 1.48 (s, 18H), 1.21 (s, 7H).

**dimethyl (R,R,1S,1'S)-2,2'-((2S,2'S,5S,5'S)-5,5'-(5,5'-(5,10-dihydrochromeno[5,4,3-cde]chromene-2,7-diyl)bis(1H-imidazole-5,2-diyl))bis(2-methylpyrrolidine-5,1-diyl))bis(1-((2R,6R)-2,6-dimethyltetrahydro-2H-pyran-4-yl)-2-oxoethane-2,1-diyl)dicarbamate (Compound 14-5).**

To a solution of (2S,2'S,5S,5'S)-tert-butyl5,5'-(5,5'-(5,10-dihydrochromeno[5,4,3-cde]chromene-2,7-diyl)bis(1H-imidazole-5,2-diyl))bis(2-methylpyrrolidine-1-carboxylate) (0.5 g, 0.705 mmol) in a mixture of dichloromethane (3 mL) and methanol (0.5 mL) was added HCl (4 M in dioxane, 3.527 mL, 14.11 mmol). The solution was stirred at room temperature for 1 hour. Upon completion, the reaction mixture was concentrated in vacuo. The resulting solid was dissolved in DMF (3.5 mL), followed by the addition of (S)-2-((2R,6R)-2,6-dimethyltetrahydro-2H-pyran-4-yl)-2-(methoxycarbonylamino)acetic acid (0.38 g, 1.55 mmol) (Compound 14-4), HATU (0.72 g, 1.90 mmol) and diisopropylethylamine (0.86 mL, 4.93 mmol). The solution was stirred at room temperature. Upon completion by LCMS the reaction mixture was purified by reverse phase HPLC (Gemini column, 10-53% MeCN/water (0.1% TFA modifier)) and lyophilized to give dimethyl (R,R,1S,1'S)-2,2'-((2S,2'S,5S,5'S)-5,5'-(5,5'-(5,10-dihydrochromeno[5,4,3-cde]chromene-2,7-diyl)bis(1H-imidazole-5,2-diyl))bis(2-methylpyrrolidine-5,1-diyl))bis(1-((2R,6R)-2,6-dimethyltetrahydro-2H-pyran-4-yl)-2-oxoethane-2,1-diyl)dicarbamate (0.395 g, 58%) as a white powder. LCMS-ESI+ calc'd for $C_{52}H_{67}N_8O_{10}$: 963.49 (M+); Found: 963.51(M+1). $^1H$ NMR (400 MHz, Methanol-d4) $\delta$ 7.97 - 7.85 (m, 2H), 7.38 - 7.19 (m, 3H), 5.43 - 5.29 (m, 4H), 5.22 - 5.08 (m, 2H), 4.80 - 4.73 (m, 1H), 4.35 - 4.18 (m, 3H), 4.13 (d, J = 9.2 Hz, 2H), 3.73 (s, 2H), 3.66 (s, 5H), 2.57 - 2.47 (m, 2H), 2.44 - 2.14 (m, 7H), 2.06 - 1.93 (m, 2H), 1.77 - 1.61 (m, 2H), 1.55 (d, J = 6.6 Hz, 5H), 1.50 - 1.38 (m, 2H), 1.35 - 1.28 (m, 2H), 1.22 (d, J = 6.9 Hz, 4H), 1.18 - 1.09 (m, 3H), 1.04 (d, J = 6.1 Hz, 4H), 1.01 - 0.90 (m, 2H).

**Example 15**

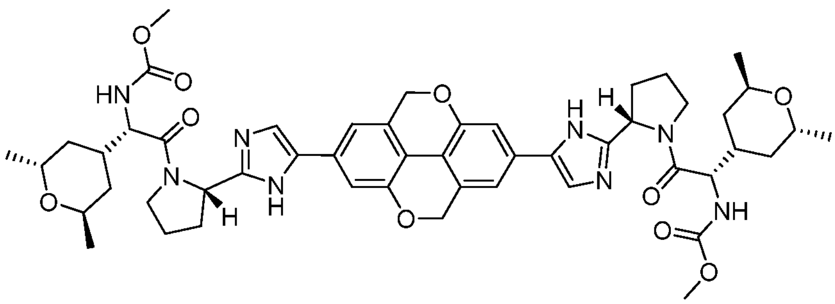

**dimethyl ((1S,1'S)-((2S,2'S)-((5,10-dihydrochromeno[5,4,3-cde]chromene-2,7-diyl)bis(1H-imidazole-5,2-diyl))bis(pyrrolidine-2,1-diyl))bis(1-((2R,6R)-2,6-dimethyltetrahydro-2H-pyran-4-yl)-2-oxoethane-2,1-diyl))dicarbamate (Compound 15).**

Following Example 14, substituting ((2S,5S)-1-(tert-butoxycarbonyl)-5-methylpyrrolidine-2-carboxylic acid with (S)-1-(tert-butoxycarbonyl)pyrrolidine-2-carboxylic acid provided dimethyl (R,R,1S,1'S)-2,2'-((2S,2'S)-2,2'-(5,5'-(5,10-dihydrochromeno[5,4,3-cde]chromene-2,7-diyl)bis(1H-imidazole-5,2-diyl))bis(pyrrolidine-2,1-diyl))bis(1-((2R,6R)-2,6-dimethyltetrahydro-2H-pyran-4-yl)-2-oxoethane-2,1-diyl)dicarbamate (0.315g, 46%) as a white powder. LCMS-ESI+ calc'd for $C_{50}H_{63}N_8O_{10}$: 935.47; Found: 935.579. $^1H$ NMR (400 MHz, Methanol-d4) $\delta$ 7.89 (s, 3H), 7.24 (d, J = 11.5, 1.5 Hz, 4H), 5.34 (s, 4H), 5.23 (t, J = 7.4 Hz, 2H), 4.29 - 4.05 (m, 6H), 3.96 - 3.82 (m, 3H), 3.78 - 3.60 (m, 7H), 2.65 - 2.47

(m, 3H), 2.41 - 2.03 (m, 9H), 1.65 - 1.38 (m, 5H), 1.32 - 1.25 (m, 3H), 1.21 (d, J = 6.8 Hz, 5H), 1.13 (d, J = 6.2 Hz, 1H), 1.05 (d, J = 6.1 Hz, 5H), 0.97 (q, J = 12.0 Hz, 2H).

**Example 16**

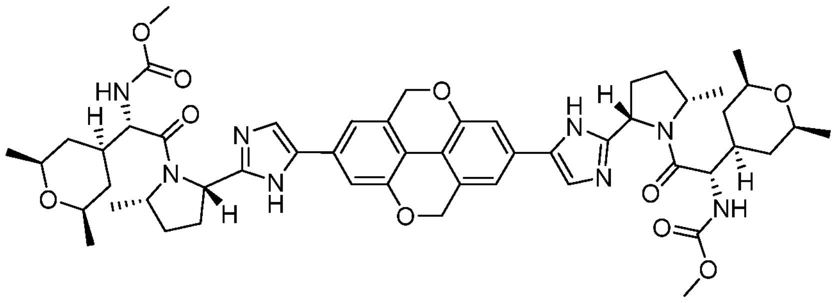

**dimethyl (r,S,R,1S,1'S)-2,2'-((2S,2'S,5S,5'S)-5,5'-(5,5'-(5,10-dihydrochromeno[5,4,3-cde]chromene-2,7-diyl)bis(1H-imidazole-5,2-diyl))bis(2-methylpyrrolidine-5,1-diyl))bis(1-((2R,4r,6S)-2,6-dimethyltetrahydro-2H-pyran-4-yl)-2-oxoethane-2,1-diyl)dicarbamate (Compound 16).**

Following Example 14, substituting (S)-2-((2R,6R)-2,6-dimethyltetrahydro-2H-pyran-4-yl)-2-(methoxycarbonylamino)acetic acid with (S)-2-((2R,4r,6S)-2,6-dimethyltetrahydro-2H-pyran-4-yl)-2-(methoxycarbonylamino)acetic acid provided dimethyl (r,S,R,1S,1'S)-2,2'-((2S,2'S,5S,5'S)-5,5'-(5,5'-(5,10-dihydrochromeno[5,4,3-cde]chromene-2,7-diyl)bis(1H-imidazole-5,2-diyl))bis(2-methylpyrrolidine-5,1-diyl))bis(1-((2R,4r,6S)-2,6-dimethyltetrahydro-2H-pyran-4-yl)-2-oxoethane-2,1-diyl)dicarbamate (0.157g, 58%) as a white powder. LCMS-ESI+ calc'd for $C_{52}H_{67}N_8O_{10}$: 963.49; Found: 963.398. $^1$H NMR (400 MHz, Methanol-d4) $\delta$ 7.99 - 7.77 (m, 2H), 7.54 - 7.14 (m, 4H), 5.43 - 5.28 (m, 4H), 5.13 (dd, J = 10.5, 7.2 Hz, 1H), 4.76 (t, J = 7.2 Hz, 2H), 4.35 - 4.10 (m, 2H), 3.80 - 3.62 (m, 5H), 3.56 - 3.37 (m, 2H), 2.56 - 2.46 (m, 1H), 2.43 - 2.17 (m, 4H), 2.11 - 1.91 (m, 3H), 1.87 - 1.68 (m, 2H), 1.54 (d, J = 6.7 Hz, 5H), 1.26 (d, J = 6.2 Hz, 1H), 1.21 - 1.01 (m, 14H), 0.92 (p, J = 11.6 Hz, 4H).

**BIOLOGICAL ASSAYS**

**Effect of serum proteins on replicon potency:** Replicon assays are conducted in normal cell culture medium (DMEM + 10%FBS) supplemented with physiologic concentrations of human serum albumin (40 mg/mL) or $\alpha$-acid glycoprotein (1 mg/mL). $EC_{50}$ in the presence of human serum proteins are compared to the $EC_{50}$ in normal medium to determine the fold shift in potency.

**MT-4 Cell Cytotoxicity:** MT4 cells are treated with serial dilutions of compounds for a five day period. Cell viability is measured at the end of the treatment period using the Promega CellTiter-Glo assay and non-linear regression is performed to calculate $CC_{50}$.

**Compound Concentration Associated with Cells at $EC_{50}$:** Huh-luc cultures are incubated with compound at concentrations equal to $EC_{50}$. At multiple time points (0 - 72 hours), cells are washed 2X with cold medium and extracted with 85% acetonitrile; a sample of the media at each time-point will also be extracted. Cell and media extracts are analyzed by LC/MS/MS to determine the Molar concentration of compounds in each fraction. Representative compounds described herein have shown activity.

**Solubility and Stability:** Solubility is determined by taking an aliquot of 10 mM DMSO stock solution and preparing the compound at a final concentration of 100 $\mu$M in the test media solutions (PBS, pH 7.4 and 0.1 N HCl, pH 1.5) with a total DMSO concentration of 1%. The test media solutions are incubated at room temperature with shaking for 1 hr. The solutions will then be centrifuged and the recovered supernatants are assayed on the HPLC/UV. Solubility will be calculated by comparing the amount of compound detected in the defined test solution compared to the amount detected in DMSO at the same concentration. Stability of compounds after an 1 hour incubation with PBS at 37°C will also be determined.

**Stability in Cryopreserved Human, Dog, and Rat Hepatocytes:** Each compound is incubated for up to 1 hour in hepatocyte suspensions (100 $\mu$L, 80,000°Cells per well) at 37°C. Cryopreserved hepatocytes are reconstituted in the serum-free incubation medium. The suspension is transferred into 96-well plates (50 $\mu$L/well). The compounds are diluted to 2 $\mu$M in incubation medium and then are added to hepatocyte suspensions to start the incubation. Samples are taken at 0, 10, 30 and 60 minutes after the start of incubation and reaction will be quenched with a mixture consisting

of 0.3% formic acid in 90% acetonitrile/10% water. The concentration of the compound in each sample is analyzed using LC/MS/MS. The disappearance half-life of the compound in hepatocyte suspension is determined by fitting the concentration-time data with a monophasic exponential equation. The data will also be scaled up to represent intrinsic hepatic clearance and/or total hepatic clearance.

**Stability in Hepatic S9 Fraction from Human, Dog, and Rat:** Each compound is incubated for up to 1 hour in S9 suspension (500 μl, 3 mg protein/mL) at 37°C (n = 3). The compounds are added to the S9 suspension to start the incubation. Samples are taken at 0, 10, 30, and 60 minutes after the start of incubation. The concentration of the compound in each sample is analyzed using LC/MS/MS. The disappearance half-life of the compound in S9 suspension is determined by fitting the concentration-time data with a monophasic exponential equation.

**Caco-2 Permeability:** Compounds are assayed via a contract service (Absorption Systems, Exton, PA). Compounds are provided to the contractor in a blinded manner. Both forward (A-to-B) and reverse (B-to-A) permeability will be measured. Caco-2 monolayers are grown to confluence on collagen-coated, microporous, polycarbonate membranes in 12-well Costar TRANSWELL® plates. The compounds are dosed on the apical side for forward permeability (A-to-B), and are dosed on the basolateral side for reverse permeability (B-to-A). The cells are incubated at 37°C with 5% $CO_2$ in a humidified incubator. At the beginning of incubation and at 1 hr and 2 hr after incubation, a 200-μL aliquot is taken from the receiver chamber and replaced with fresh assay buffer. The concentration of the compound in each sample is determined with LC/MS/MS. The apparent permeability, Papp, is calculated.

**Plasma Protein Binding:** Plasma protein binding is measured by equilibrium dialysis. Each compound is spiked into blank plasma at a final concentration of 2 μM. The spiked plasma and phosphate buffer is placed into opposite sides of the assembled dialysis cells, which will then be rotated slowly in a 37°C water bath. At the end of the incubation, the concentration of the compound in plasma and phosphate buffer is determined. The percent unbound is calculated using the following equation:

$$\%\ \text{Unbound} = 100 \bullet \left( \frac{C_f}{C_b + C_f} \right)$$

Where $C_f$ and $C_b$ are free and bound concentrations determined as the post-dialysis buffer and plasma concentrations, respectively.

**CYP450 Profiling:** Each compound is incubated with each of 5 recombinant human CYP450 enzymes, including CYP1A2, CYP2C9, CYP3A4, CYP2D6 and CYP2C19 in the presence and absence of NADPH. Serial samples will be taken from the incubation mixture at the beginning of the incubation and at 5, 15, 30, 45 and 60 minutes after the start of the incubation. The concentration of the compound in the incubation mixture is determined by LC/MS/MS. The percentage of the compound remaining after incubation at each time point is calculated by comparing with the sampling at the start of incubation.

**Stability in Rat, Dog, Monkey and Human Plasma:** Compounds will be incubated for up to 2 hours in plasma (rat, dog, monkey, or human) at 37°C. Compounds are added to the plasma at final concentrations of 1 and 10 μg/mL. Aliquots are taken at 0, 5, 15, 30, 60, and 120 minutes after adding the compound. Concentration of compounds and major metabolites at each time point are measured by LC/MS/MS.

**Evaluation of cell-based anti-HCV activity:** Antiviral potency ($EC_{50}$) was determined using a *Renilla* luciferase (RLuc)-based HCV replicon reporter assay. To perform the assay for genotype 1 and 2a JFH-1, stable HCV 1a RLuc replicon cells (harboring a dicistronic genotype 1a H77 replicon that encodes a RLuc reporter), stable HCV 1b RLuc replicon cells (harboring a dicistronic genotype 1b Con1 replicon that encodes a RLuc reporter), or stable HCV 2a JFH-1 Rluc replicon cells (harboring a dicistronic genotype 2a JFH-1 replicon that encodes a RLuc reporter; with L31 present in NS5A) were dispensed into 384-well plates for $EC_{50}$ assays. To perform the assay for genotype 2a (with M31 present in NS5A) or 2b, NS5A chimeric genotype 2a JFH-1 replicons that encodes a RLuc-Neo reporter and either genotype 2a J6 strain NS5A gene or genotype 2b MD2b-1 strain NS5A gene (both with M31 present) respectively, were either transiently transfected (t) into Huh-Lunet cells or were established as stably replicating replicon cells (s) is provided. Either cells were dispensed into 384-well plates for $EC_{50}$ assays. To perform the assay for genotype 3 and 4, NS5A chimeric genotype 1b Con1 replicons that encodes a Pi-RLuc reporter and either genotype 3a S52 strain NS5A gene or genotype 4a ED43 strain NS5A gene respectively, were transiently transfected (t) into Huh-Lunet cells, which were subsequently dispensed into 384-well plates. Compounds were dissolved in DMSO at a concentration of 10 mM and diluted in DMSO either manually or using an automated pipeting instrument. Serially 3-fold diluted compounds were either manually mixed with cell culture media and added to the seeded cells or directly added to the cells using an automated instrument. DMSO was used as a negative (solvent; no inhibition) control, and the protease inhibitor ITMN-191 was included at a concentration > 100 x $EC_{50}$ as a positive control. 72 hours later, cells were lysed and *Renilla* luciferase activity quantified as recommended by the manufacturer (Promega-Madison, WI). Non-linear regression was performed to calculate $EC_{50}$ values.

To determine the antiviral potency ($EC_{50}$) against resistance mutants, resistance mutations, including Q30R, Q30E, Y93H, and Y93N in genotype 1a NS5A, and L31V/Y93H in genotype 1b NS5A were introduced individually into either 1a Pi-Rluc or 1b Pi-Rluc replicons by site directed mutagenesis. Replicon RNA of each resistant mutant was transiently transfected into Huh-7-derived cured-51 cells and antiviral potency was determined on these transfected cells as described above.

**IV and PO Single Dose Pharmacokinetic Studies in SD Rats:** The pharmacokinetics of selected compounds was characterized in male Sprague-Dawley (SD) rats (250-300 g). In this study, two groups of naive purebred SD rats (N=3 per group, fasted over night) received the selected compound either as an intravenous (IV) infusion (1 mg/kg over 30 minutes) via the jugular vein or by oral gavage (2 mg/kg). The intravenous (IV) dosing vehicle was 5% ethanol, 35% polyethylene glycol 400 (PEG 400) and 60% water pH 2.0. The oral dosing vehicle was 5% ethanol, 55% PEG 400 and 40% citrate buffer pH 2.2.

Serial blood samples (approximately 0.3 mL each) were collected from jugular vein or other suitable vein at specified time points. For the IV infusion group, the blood samples were collected predose and at 0.25, 0.48, 0.58, 0.75, 1.5, 3, 6, 8, 12 and 24 hours after the start of infusion. For the oral group, the blood samples were collected predose and at 0.25, 0.50, 1, 2, 4, 6, 8, 12 and 24 hours after dosing. The blood samples were collected into Vacutainer™ tubes containing EDTA-$K_3$ as the anti-coagulant and were centrifuged at approximately 4 °C to obtain plasma. The plasma samples were stored at -20°C until analysis by LC/MS/MS.

A bioanalytical method utilizing high performance liquid chromatography coupled to tandem mass spectrometry (LC/MS/MS) was developed for analysis of the selected compound in rat plasma. Detection was performed using selected reaction monitoring (SRM); Ions representing the precursor $(M+H)^+$ species was selected in quadrupole 1 (Q1) and collided with argon gas in the collision cell (Q2) to generate specific product ion, which was subsequently monitored by quadrupole 3 (Q3). Standard curve and quality control samples were prepared in male rat plasma and processed in the same way as the test samples to generate quantitative data.

Pharmacokinetic parameters were generated using non-compartmental pharmacokinetic analysis (Phoenix WinNonlin, version 6.3). Values below the lower limit of quantification (LLOQ) were assigned a value of zero if predose and treated as missing thereafter. Area under the curve (AUC) was calculated using the linear trapezoidal rule. The oral bioavailability (%F) was determined by comparison of the area under the curve (AUC) of the compound and/or a metabolite generated in plasma following oral administration to that generated following intravenous administration.

Data obtained in the above described assays for the compounds as described herein is shown in Table 1.

**Table 1**

| Compound No. | 1b (nM) | 1a (nM) | 2a JFH (nM) | 2a J6 (t) (nM) | 2b (t) (nM) | 2b (s) (nM) | 3a (nM) | 4a (s) (nM) |
|---|---|---|---|---|---|---|---|---|
| 14 | 0.054 | 0.049 | 0.011 | 0.014 | 0.038 | | 0.04 | 0.011 |
| 15 | 0.195 | 0.18 | 0.024 | | | 0.143 | 0.199 | 0.07 |
| 16 | 0.037 | 0.043 | 0.013 | | | 0.054 | 0.051 | 0.025 |
| **Compound No.** | **Rat %F** | **1b L31V/ Y93H (nM)** | **1a Q30R (nM)** | **1a Q30E (nM)** | **1a Y93H (nM)** | **1a Y93N (nM)** | **3a Y93H (nM)** | |
| 14 | 15 | 0.075 | | 0.110 | 0.063 | 0.229 | 0.157 | |
| 15 | | | | | 0.237 | 0.870 | 0.361 | |
| 16 | | | | | 0.356 | 0.685 | 0.145 | |

## Claims

1. A compound having the formula:

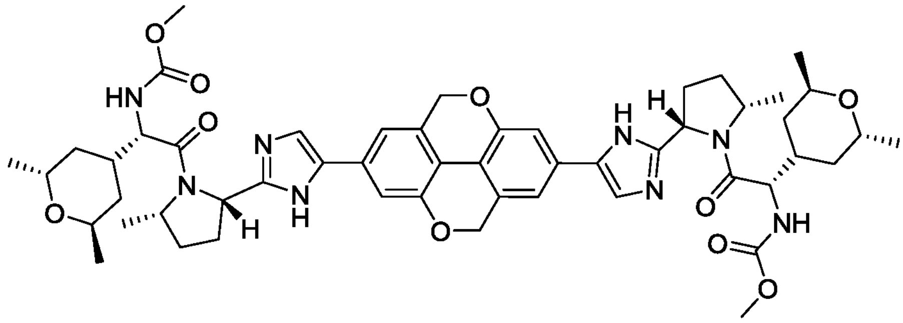

,

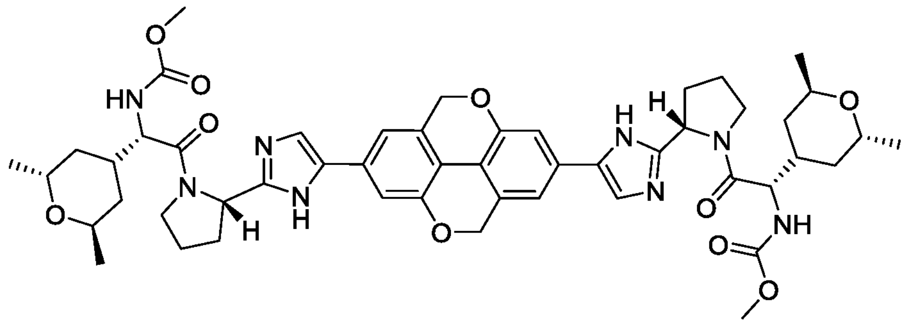

,

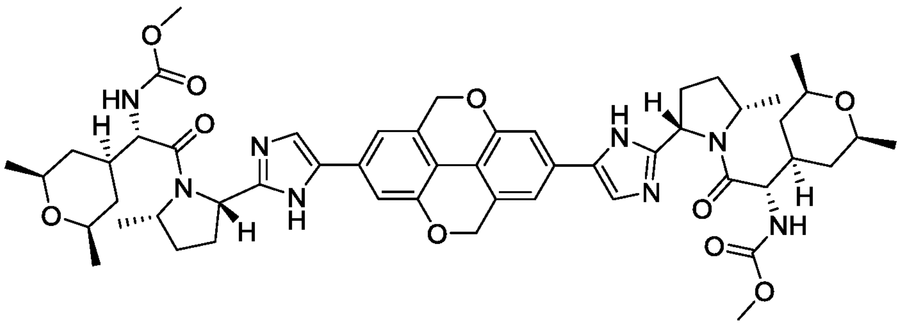

or a pharmaceutically acceptable salt thereof.

2. A compound or salt according to claim 1, for use in treating HCV.

3. A pharmaceutical composition comprising a compound of claim 1 and at least one pharmaceutically acceptable carrier.

4. A pharmaceutical composition according to claim 3, further comprising at least one additional therapeutic agent for treating HCV.

5. A pharmaceutical composition according to claim 3, further comprising at least one inhibitor of HCV NS5B polymerase.

6. A pharmaceutical composition according to claim 5, wherein the inhibitor of HCV NS5B polymerase is sofosbuvir.

7. A pharmaceutical composition according to claim 3, for use in treating HCV.

## Patentansprüche

1. Verbindung der Formel:

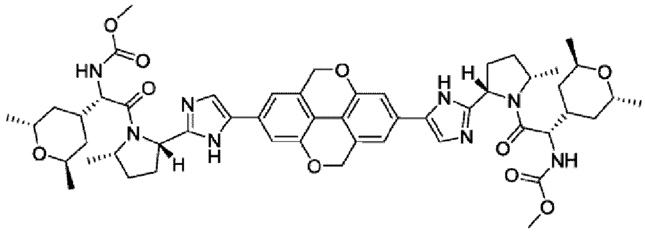

,

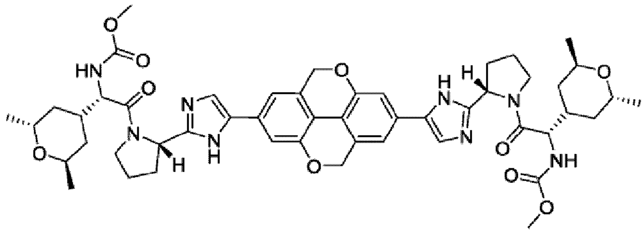

,

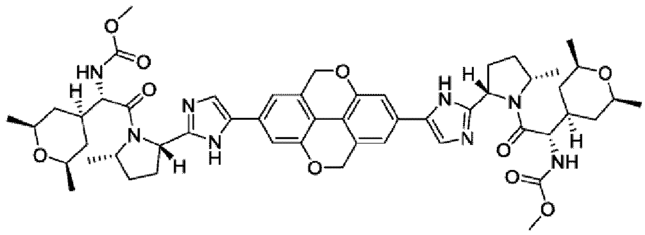

oder ein pharmazeutisch verträgliches Salz davon.

2. Verbindung oder Salz nach Anspruch 1 zur Verwendung bei der Behandlung von HCV.

3. Pharmazeutische Zusammensetzung umfassend eine Verbindung nach Anspruch 1 und mindestens einen pharmazeutisch verträglichen Träger.

4. Pharmazeutische Zusammensetzung nach Anspruch 3, ferner umfassend mindestens ein zusätzliches therapeutisches Mittel zur Behandlung von HCV.

5. Pharmazeutische Zusammensetzung nach Anspruch 3, ferner umfassend mindestens einen HCV-NS5B-Polymerasehemmer.

6. Pharmazeutische Zusammensetzung nach Anspruch 5, wobei der HCV-NS5B-Polymerasehemmer Sofosbuvir ist.

7. Pharmazeutische Zusammensetzung nach Anspruch 3 zur Verwendung bei der Behandlung von HCV.

## Revendications

1. Composé ayant la formule

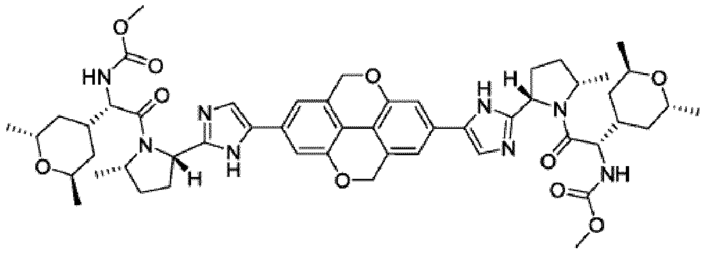

,

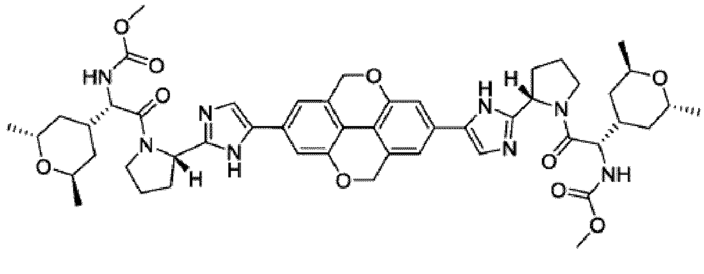

,

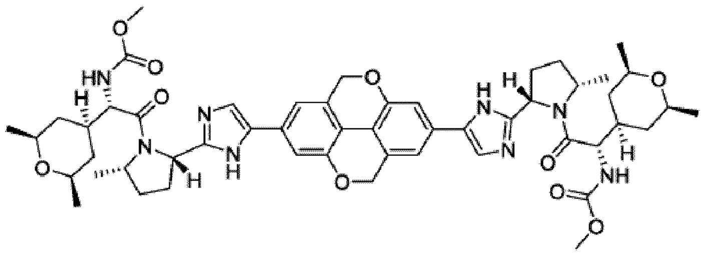

ou un sel pharmaceutiquement acceptable de celui-ci.

2. Composé ou sel selon la revendication 1, pour une utilisation dans le traitement du VHC.

3. Composition pharmaceutique comprenant un composé selon la revendication 1 et au moins un excipient pharmaceutiquement acceptable.

4. Composition pharmaceutique selon la revendication 3, comprenant en outre au moins un agent thérapeutique supplémentaire pour traiter le VHC.

5. Composition pharmaceutique selon la revendication 3, comprenant en outre au moins un inhibiteur de la polymérase NS5B du VHC.

6. Composition pharmaceutique selon la revendication 5, dans laquelle l'inhibiteur de la polymérase NS5B du VHC est le sofosbuvir.

7. Composition pharmaceutique selon la revendication 3, pour une utilisation dans le traitement du VHC.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013185093 A
- US 20130102525 A
- WO 2014008285 A


### Non-patent literature cited in the description


- HCV NS5A for genotypes 1a and 1b. *Antimicrobial Agents and Chemotherapy,* September 2010, vol. 54, 3641-3650
- Handbook of Pharmaceutical Excipients. 1986
- Remington's Pharmaceutical Sciences. Mack Publishing Co,